# EUROPEAN PATENT SPECIFICATION

(11) **EP 3 783 367 B1**
(45) Date of publication and mention of the grant of the patent: **29.11.2023**
(21) Application number: 19788429.9
(22) Date of filing: 28.01.2019
(51) Int. Cl.: G01N 35/10, B01L 3/00

(54) **SAMPLING MECHANISM AND SAMPLING METHOD**
PROBENAHMEVORRICHTUNG UND PROBENAHMEVERFAHREN
MÉCANISME D'ÉCHANTILLONNAGE ET PROCÉDÉ D'ÉCHANTILLONNAGE

(30) Priority: 20.04.2018 JP 2018081134
(43) Date of publication of application: 24.02.2021
(73) Proprietor: Sekisui Medical Co., Ltd., Tokyo 103-0027 (JP)
(72) Inventor: KAWABE, Toshiki, Tokyo 103-0027 (JP)
(74) Representative: Schäfer, Matthias W.
(86) International application number: PCT/JP2019/002649
(87) International publication number: WO 2019/202805

(56) References cited:
- WO-A1-2017/018214
- WO-A1-2017/018214
- DE-U1- 20 001 817
- JP-A- H0 783 938
- JP-A- H0 783 938
- JP-A- 2004 003 916
- JP-A- 2004 003 916
- US-A1- 2007 095 159

## Description

### TECHNICAL FIELD

The present invention relates to a sampling mechanism and a sampling method capable of collecting a sample such as blood for analysis from a container sealed with a plug.

### BACKGROUND ART

For example, some automatic analysis devices such as blood coagulation analysis devices employ closed tube sampling (CTS) for sampling a sample with a plug (hereinafter, also referred to as a cap) attached to a blood collection tube (Patent Document 1 and so on). A needle-shaped piercer that is a hollow tube is used for this CTS. Further, a hole is formed in the plug by the piercer (the plug is perforated) and a sample probe reaches the inside of the blood collection tube through the inside of the piercer and collects the sample. It should be noted that the CTS sampling method is sometimes called a cap piercing method or the like.

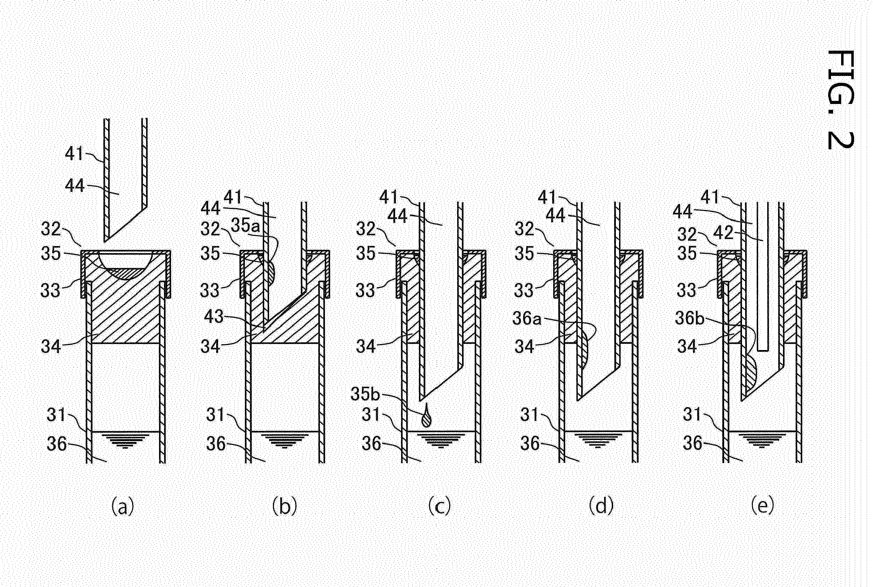

Figs. 2(a) to 2(e) schematically illustrate a typical CTS procedure from perforation (piercing) to sampling. A piercer 41 illustrated in Fig. 2(a) is a pointed cylindrical member in which an open tip is diagonally processed. In an analysis device (or an inspection device or sampling device) provided with the piercer 41, a sample container (here, a blood collection tube 31) to be sampled is disposed at a predetermined sampling position.

Further, as illustrated in Figs. 2(a) and 2(b), the piercer 41 descends from above the blood collection tube 31 and is inserted into a cap 32 mounted on the blood collection tube 31. In the example illustrated in Figs. 2 (a) and 2 (b), the cap 32 is configured to include a cap main body 33 that is a rigid plastic body and a perforated portion 34 that is an elastic body such as rubber and fitted in the middle portion of the cap main body 33.

Further, as illustrated in Fig. 2(b), the piercer 41 pierces the perforated portion 34 and descends while widening a hole 43 of the perforated portion 34. Then, the piercer 41 penetrates the perforated portion 34 as illustrated in Fig. 2 (c) and stops as illustrated in Fig. 2(d). As a result, a space 44 is ensured in the perforated portion 34 as illustrated in Fig. 2(d).

Subsequently, a sample probe (hereinafter, referred to as "probe") 42 descends from above the stopped piercer 41 and enters the blood collection tube 31 through the space 44 of the piercer 41 as illustrated in Fig. 2 (e) . Further, although not illustrated, the lower end portion (tip portion) of the probe 42 reaches and stops inside the blood collection tube 31 and a sample (plasma) 36 in the blood collection tube 31 is suctioned via the probe 42. Then, the probe 42 moves to a predetermined ejection position and the sample 36 is ejected.

### CITATION LIST

### PATENT DOCUMENT

Patent Document 1: JP 2016-176774 A
Patent Document 2: JP 2929406 B2
US 2007/095159 A1 discloses a sampling mechanism for inserting a sampling double needle into a tube stopped by a bung. In particular, it discloses a sampling mechanism for inserting a tubular perforating body into a plug body of a sampled container accommodating a sample and performing sample sampling by passing a probe through the perforating body with the plug body penetrated, the sampling mechanism comprising the perforating body, wherein the perforating body has: a first perforating body inserted into the plug body and penetrating the plug body; and a second perforating body, approaching the plug body together with the first perforating body by causing the first perforating body to protrude downward, reaching the plug body after the first perforating body reaches the plug body. DE 200 01 817 U1 discloses a piercing device having a double canula for the sterile removal of a liquid medium from a container having a pierceable closures container. JPH07 83938 A discloses a nozzle part constituted of an inner nozzle and an outer nozzle, wherein for sampling a sample, the inner nozzle is housed inside the outer nozzle while piercing the nozzle part into a rubber stopper. JP 2004 003916 A discloses a specimen suction tube for penetrating a plug sealing a sealed specimen vessel with a specimen stored inside, wherein the specimen suction tube is constituted of a small-diameter pipe disposed inside a large-diameter pipe. WO 2017/018214 A1 and US 2018/210003 A1 disclose a pierce needle for forming a cut in a reagent bottle lid having an expansion portion pushing and opening a cut as well as a piercing portion forming the cut which are integrated with each other.

### SUMMARY OF THE INVENTION

### PROBLEM TO BE SOLVED BY THE INVENTION

By the way, during the CTS-based sample collection as described above, sampling is performed with the blood collection tube 31 sealed with the cap 32, and thus it is excellent in various points such as infection prevention for a worker who performs blood collection or inspection and prevention of evaporation of blood as a contained body. However, as illustrated in Fig. 2(a), blood 35 may, for example, be attached to the upper surface of the cap 32 during or after blood collection (vacuum blood collection).

Although not illustrated, during blood collection, the blood collection tube 31 is mounted with respect to a blood collection tube holder to which a blood collection needle is attached (or connected to the blood collection needle via a tube). At this time, the needle covered with sheath rubber protruding into the blood collection tube holder pierces the perforated portion 34 of the blood collection tube 31. The barometric pressure inside the blood collection tube 31 is a negative pressure in accordance with a prescribed blood collection amount before blood collection, and the blood of a subject flows into the blood collection tube by means of the pressure difference from the venous pressure of the subject.

When the blood collection tube 31 is removed from the blood collection tube holder after blood collection, blood leaking from the needle exposed until the contracted sheath rubber returns to its original state may adhere to the upper surface of the cap 32.

As described above, the blood 35 may adhere to the upper surface of the cap 32. When the blood 35 is measured as it is without being removed, blood 35a may enter the piercer 41 and adhere to the inner wall surface of the piercer 41 when the piercer 41 enters the perforated portion 34 as illustrated in Fig. 2(b). Then, the blood 35 outside the cap 32 may be brought to the inside of the cap 32 via the piercer 41 and fall into the sample 36 as indicated by reference numeral 35b in Fig. 2 (c) . In addition, a negative pressure may remain in the blood collection tube 31 in a case where, for example, the amount of blood collection during blood collection is smaller than a specified value . Further, in such a case, the blood 35 is highly likely to enter the blood collection tube 31 when the CTS is used and the sample 36 is highly likely to be contaminated.

In addition, when the piercer 41 enters the perforated portion 34 or descends, the perforated portion 34 is pushed toward the inside of the blood collection tube 31 by the piercer 41 and elastically deformed to some extent. Then, the air inside the blood collection tube 31 is compressed by the elastic deformation of the perforated portion 34 and the barometric pressure inside the blood collection tube 31 is increased. Then, when the piercer 41 penetrates the perforated portion 34, the inside and the outside of the blood collection tube 31 are connected via the space 44 of the piercer 41.

Accordingly, when the piercer 41 penetrates the perforated portion 34, the increased pressure in the blood collection tube 31 is released and the sample 36 may be scattered due to an instantaneous change in pressure. Further, due to the scattering of the sample 36, a scattered sample 36a may adhere to the inner wall surface of the piercer 41 and a sample 36b may result from so-called dripping (Fig. 2(e)).

Further, in a case where the analysis device is, for example, of a type that automatically suctions the sample 36 when the liquid surface of the sample 36 is detected, the surface of the sample 36b attributable to dripping may be erroneously recognized as an appropriate liquid surface and suction by the probe 42 may be started when the probe 42 comes into contact with the sample 36b attributable to the dripping as illustrated in Fig. 2(e). However, even if the sample 36b attributable to the dripping is suctioned, the amount of suction is not sufficient for analysis and so-called empty suction occurs. Then, such empty suction leads to a decline in the accuracy of inspection using the analysis device. It should be noted that such empty suction may also occur in the blood 35a attached inside the piercer 41.

Further, after syringe-collected blood is injected into the blood collection tube 31 with the cap 32 removed, the inside of the blood collection tube plugged with the cap 32 has a positive pressure, and thus the sample 36 may be scattered out of the blood collection tube through the space 44 of the piercer 41 during CTS. Further, in such a case, a worker who has opened the cover of the analysis device may touch the blood scattered in the analysis device or the scattered blood 35 and sample 36 may contaminate the surrounding parts in the analysis device (equipment contamination).

In addition, in a case where the piercer 41 does not properly pierce the perforated portion 34 in descending, the piercer 41 may continue to push the perforated portion 34, separate the perforated portion 34 from the cap main body 33, and be pushed into the blood collection tube 31. Further, when the probe 42 descends in such a case, the pushed perforated portion 34 hinders the entry of the probe 42 into the sample 36 and normal sampling cannot be performed.

As described above, the CTS of the related art may entail the above problems depending on the situation. Then, it is conceivable that the problems lead to deterioration in the accuracy of inspection using the analysis device. Accordingly, it is necessary to take measures to forestall the various situations described above.

The invention has been made to solve such problems, and an object thereof is to provide a sampling mechanism and a sampling method capable of performing sampling through proper perforation with respect to a plug body.

### MEANS FOR SOLVING PROBLEM

(1) In order to achieve the above object, the invention provides a sampling mechanism for inserting a tubular perforating body into a plug body of a sampled container and performing sample sampling by passing a probe through the perforating body with the plug body penetrated, the sampling mechanism comprising the perforating body, wherein the perforating body has:
   a first perforating body inserted into the plug body and penetrating the plug body with a tip closed; and
   a second perforating body formed in a tubular shape, disposed outside the first perforating body, inside of which the first perforating body is slidably mounted with extremely slight gap, and approaching the plug body together with the first perforating body by causing the first perforating body to protrude downward, reaching the plug body after the first perforating body reaches the plug body, and penetrating the plug body by being inserted into the plug body, and
   the probe is passed through the second perforating body remaining after retraction of the first perforating body.
(2) In addition, in order to achieve the above object, another invention provides the sampling mechanism in which the first perforating body has a ventilation passage open toward a side portion at a part reaching an internal space of the sampled container and allowing an inside and an outside of the sampled container to communicate with each other.
(3) In addition, in order to achieve the above object, another invention provides an automatic analysis device including the sampling mechanism according to (1) or (2).
(4) In addition, in order to achieve the above object, another invention provides a sampling method for inserting a perforating body into a plug body of a sampled container and performing sample sampling by passing a probe through the perforating body, the perforating body having a first perforating body having a closed tip and a second perforating body formed in a tubular shape, disposed outside the first perforating body, inside of which the first perforating body is slidably mounted with extremely slight gap, the sampling method including:
   a step of disposing the second perforating body outside the first perforating body, causing the first perforating body to protrude below the second perforating body, and causing the first perforating body and the second perforating body to approach the plug body together;
   a step of inserting the first perforating body into the plug body;
   a step of causing the second perforating body to reach the plug body after the first perforating body reaches the plug body and inserting the second perforating body into the plug body;
   a step of causing the first perforating body and the second perforating body to penetrate the plug body;
   a step of retracting the first perforating body; and
   a step of passing the probe through the remaining second perforating body.
(5) In addition, in order to achieve the above object, another invention provides the sampling method in which the first perforating body is provided with a ventilation passage open toward a side portion at a part reaching an internal space of the sampled container and the first perforating body is retracted after the ventilation passage allows an inside and an outside of the sampled container to communicate with each other.

### EFFECT OF THE INVENTION

According to the above configuration, it is possible to provide a sampling mechanism and a sampling method capable of performing sampling through proper perforation with respect to a plug body.

### BRIEF DESCRIPTION OF DRAWINGS

Figs. 1(a) to 1(e) are explanatory views sequentially illustrating the operation of perforation in a sampling mechanism according to an embodiment of the invention; and
Figs. 2(a) to 2(e) are explanatory views sequentially illustrating the operation of perforation in a sampling mechanism according to the related art.

### MODE(S) FOR CARRYING OUT THE INVENTION

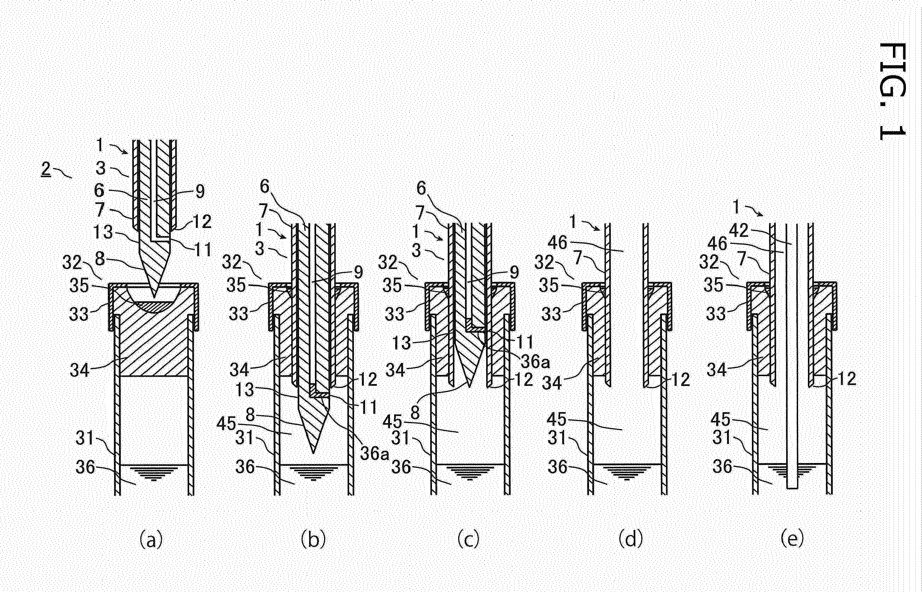

Hereinafter, a sampling mechanism according to an embodiment of the invention and a sampling method executed by means of this sampling mechanism will be described with reference to the drawings. It should be noted that parts similar to those of the background art illustrated in Fig. 2 are denoted by the same reference numerals and description thereof are appropriately omitted. Fig. 1(a) illustrates a sampling mechanism 1 according to the present embodiment. Although not illustrated in detail, this sampling mechanism 1 is provided in a sampling unit of an automatic analysis device 2.

Here, the automatic analysis device 2 dispenses each of a sample to be analyzed and a reagent to be mixed with the sample into a reaction container. Further, although not illustrated, the automatic analysis device 2 includes, for example, a measuring unit optically measuring the reaction that occurs in the reaction container, a rack transport unit transporting a rack holding a sample container to the measuring unit, and a control unit performing overall control including the measuring unit and the rack transport unit. Further, the automatic analysis device 2 is capable of automatically performing biochemical, immunological, hematological, or genetic sample analysis by linking these component parts.

The sampling mechanism 1 is provided with a composite piercer 3. This composite piercer 3 is configured by a first piercer 6 as a first perforating body and a second piercer 7 as a second perforating body. Of these, the first piercer 6 has a columnar form with its axial center oriented in an up-down direction. Further, the first piercer 6 has a tapered portion 8 at its lower end and the tapered portion 8 becomes thin toward its tip side. The tapered portion 8 has the shape of a needle (or a cone).

In addition, the first piercer 6 has a ventilation passage 9 therein and the ventilation passage 9 is a space extending along the axial center. This ventilation passage 9 is formed so as to be radially bent in its lower end portion, and an opening 11 that has a circular shape or the like is formed in the side surface of the first piercer 6. Here, although the part that is above the first piercer 6 is not illustrated in Fig. 1(a), the ventilation passage 9 is connected to the external space (atmosphere) . Further, the barometric pressure inside the ventilation passage 9 is atmospheric pressure. In addition, the opening 11 is positioned at a round bar-shaped part (in a non-tapered portion 13) above the first piercer 6.

The above-mentioned second piercer 7 has the form of a circular tube with its axial center oriented in the up-down direction. Further, the tip (lower end) of the second piercer 7 is processed so as to be horizontal over the entire circumference . In addition, the lower end of the second piercer 7 is chamfered over the entire circumference and a tapered surface 12 that narrows downward is formed at the lower end of the second piercer 7 . Further, in the state that is illustrated in Fig. 1(a), the second piercer 7 is coaxially disposed outside the first piercer 6 and covers most of the first piercer 6.

Here, the inner diameter of the second piercer 7 can be, for example, approximately 2 mm to 4 mm. In addition, in the present embodiment, an extremely slight gap is formed between the inner peripheral surface of the second piercer 7 and the outer peripheral surface of the first piercer 6 to the extent that blood 35 and a sample 36 hardly enter and the axial relative movement of the first piercer 6 is not hindered. Further, although the part that is above the second piercer 7 is not illustrated in Fig. 1(a), the inside of the second piercer 7 is connected to the external space and the barometric pressure inside the second piercer 7 is atmospheric pressure.

Various materials can be adopted as the material of the first piercer 6 and the second piercer 7. In the present embodiment, stainless steel (SUS) is adopted in view of the friction with the material of a perforated portion 34 and conditions such as the rigidity that is required. By adopting stainless steel as described above, the first piercer 6 and the second piercer 7 are capable of being excellent in various conditions such as rigidity, ease of cleaning, chemical resistance, and sliding characteristics with respect to the perforated portion 34.

It should be noted that synthetic resin such as engineering plastic can be adopted as the material of the first piercer 6 and the second piercer 7 insofar as the material satisfies conditions such as perforation with respect to the perforated portion 34 being possible without hindrance and sliding with the perforated portion 34 not resulting in rubber pieces (rubber scraps). In addition, it is also possible to use, for example, a material obtained by predetermined coating of ceramics or the like on the surface of stainless steel for the first piercer 6 and the second piercer 7.

The first piercer 6 and the second piercer 7 can be independently held by various movable arm mechanisms provided in the automatic analysis device 2. Various movable arm mechanisms provided in general analysis devices can be adopted as the movable arm mechanisms. For example, although a movable arm mechanism that is capable of turning horizontally and moving up and down is equipped with a probe 42 in the present embodiment, a movable arm movement that is similar in operation to the probe 42 is applicable to each of the first piercer 6 and the second piercer 7.

As illustrated in Figs. 1(a) and 1(b), in the automatic analysis device 2 of the present embodiment, combination of the movable arm mechanisms allows the first piercer 6 and the second piercer 7 to integrally descend with the first piercer 6 inserted inside the second piercer 7. Further, in the automatic analysis device 2, the first piercer 6 can be pulled out upward from the second piercer 7 with the height of the second piercer 7 maintained as illustrated in Figs. 1(b) to 1(d).

Next, the sampling method that is performed by the automatic analysis device 2 will be described. First, as illustrated in Fig. 1(a), the first piercer 6 and the second piercer 7 (composite piercer 3) move directly above a blood collection tube 31 with the first piercer 6 inserted inside the second piercer 7. In this case, the axial center (not illustrated) of the composite piercer 3 is positioned substantially on the same straight line as the axial center (not illustrated) of the blood collection tube 31. The movement of the composite piercer 3 can be integrally performed with the first piercer 6 inserted inside the second piercer 7.

In a case where the first piercer 6 and the second piercer 7 are integrally moved in the state of the composite piercer 3 as described above, the movement and cleaning of both can be simultaneously performed together when, for example, the cleaning is performed by a cleaning unit (not illustrated) provided in the automatic analysis device 2. As a result, the movement and cleaning of the first piercer 6 and the second piercer 7 as a whole can be expedited.

It should be noted that the invention is not limited thereto. The second piercer 7 may be moved directly above the blood collection tube 31 before the first piercer 6 is moved directly above the blood collection tube 31. In this case, optimal cleaning time can be individually allocated to each of the first piercer 6 and the second piercer 7.

In the state that is illustrated in Fig. 1(a), the tapered portion 8 of the first piercer 6 protrudes from the lower end of the second piercer 7 and is exposed to the outside of the second piercer 7. Further, the opening 11 of the ventilation passage 9 provided in the first piercer 6 is exposed to the outside of the second piercer 7. It should be noted that the invention is not limited thereto. The position of the first piercer 6 may be controlled such that the opening 11 is hidden by the second piercer 7 and the opening 11 may come out of and be exposed from the second piercer 7 by the first piercer 6 being further lowered after the second piercer 7 (and the first piercer 6) enters the blood collection tube 31 at the position illustrated in Fig. 1(b) so that the blood 35 is prevented from entering the opening 11 when the first piercer 6 pierces the perforated portion 34. In Fig. 1(a), the first piercer 6 and the second piercer 7 descend toward the blood collection tube 31 and the lower end of the first piercer 6 reaches the perforated portion 34 of a cap 32. At this time, the position where the tip of the first piercer 6 comes into contact is the middle portion on the upper surface of the perforated portion 34.

Further, in the state that is illustrated in Fig. 1(a), the blood 35 is attached substantially in the middle on the upper surface of the perforated portion 34. The first piercer 6 and the second piercer 7 (composite piercer 3) continue to descend, and the first piercer 6 presses the perforated portion 34. The tip of the tapered portion 8 of the first piercer 6 has a small diameter, and thus the first piercer 6 pierces the perforated portion 34 and a hole (reference numeral omitted) is formed around the tapered portion 8. Here, although a hole is also formed during blood collection, this hole formed during the blood collection is closed by the elasticity of rubber or the like after the blood collection. Further, in some cases, the first piercer 6 enters the hole formed during the blood collection.

Further, by the first piercer 6 and the second piercer 7 continuing to descend integrally, the first piercer 6 enters deep inside the perforated portion 34 while widening the hole (reference numeral omitted) of the perforated portion 34 with the tapered portion 8. Then, the amount of expansion of the hole by the first piercer 6 is maximized when the entire tapered portion 8 of the first piercer 6 enters the hole.

In addition, the second piercer 7 descends together with the first piercer 6, and thus the second piercer 7 also reaches the perforated portion 34. The tapered surface 12 is formed at the lower end of the second piercer 7 as described above, and this tapered surface 12 is positioned above the tapered portion 8 of the first piercer 6. Accordingly, the perforated portion 34 is somewhat widened by the tapered surface 12 of the second piercer 7 and the second piercer 7 enters the hole of the perforated portion 34 from the lower end. Then, the second piercer 7 descends integrally with the first piercer 6 while maintaining its positional relationship with the first piercer 6.

Here, in the present embodiment, the positional relationship between the tapered surface 12 of the second piercer 7 and the tapered portion 8 of the first piercer 6 is such that the round bar-shaped part (non-tapered portion 13) of the first piercer 6 is interposed between the two. In addition, the opening 11 of the first piercer 6 is positioned in this non-tapered portion 13.

It should be noted that the invention is not limited thereto. It is possible to dispose the tapered portion 8 and the tapered surface 12 so as to be continuous to the maximum extent possible by preventing the non-tapered portion 13 from being exposed or minimizing the exposure of the non-tapered portion 13 by bringing the tapered portion 8 and the tapered surface 12 closer to each other. Then, it is conceivable that the perforated portion 34 can be smoothly delivered from the tapered portion 8 to the tapered surface 12 as a result. In addition, although not illustrated, it is conceivable in this case to dispose the opening 11 of the first piercer 6 in the tapered portion 8.

As illustrated in Fig. 1(b), both the first piercer 6 and the second piercer 7 penetrate the perforated portion 34 when the first piercer 6 and the second piercer 7 (composite piercer 3) are further lowered. At this time, the opening 11 of the first piercer 6 reaches an internal space 45 of the blood collection tube 31 and the pressure inside the blood collection tube 31 is released via the ventilation passage 9. It should be noted that Fig. 1(b) illustrates a state where the sample 36 is scattered due to the change in pressure at the time when the perforated portion 34 is penetrated by the first piercer 6 and the second piercer 7 and a scattered sample 36a has entered the ventilation passage 9.

In this state, the automatic analysis device 2 stops the second piercer 7 and raises the first piercer 6 as illustrated in Fig. 1(c). When the first piercer 6 reaches a predetermined height, the first piercer 6 is horizontally retracted by the movable arm mechanism (not illustrated) for the first piercer 6. Then, as illustrated in Fig. 1(d), the second piercer 7 remains in a state of penetrating the perforated portion 34 and a space 46 is ensured in the perforated portion 34.

Subsequently, the probe 42 descends from above the stopped second piercer 7 and enters the blood collection tube 31 through the space 46 of the second piercer 7. Then, as illustrated in Fig. 1(e), the lower end portion (tip portion) of the probe 42 reaches and stops inside the blood collection tube 31 and the sample 36 in the blood collection tube 31 is suctioned via the probe 42. Here, in the present embodiment, the outer diameter of the probe 42 is approximately 0.8 mm to 1.5 mm.

According to the sampling mechanism 1 as described above, the first piercer 6 that performs perforation first and the second piercer 7 that performs perforation subsequently to the first piercer 6 are provided and the tip of the first piercer 6 is closed. Further, the second piercer 7 is formed with the space 46 that receives the first piercer 6 during perforation and allows the probe 42 to pass after perforation.

Accordingly, it is possible to prevent the blood 35 attached to the cap 32 from entering the first piercer 6 from the tip of the first piercer 6 during perforation by the first piercer 6. In other words, it is possible to prevent the blood 35 from being brought into the blood collection tube 31 by the first piercer 6.

Here, even if some of the blood 35 of the cap 32 is drawn into the perforated portion 34 by the tapered portion 8 of the first piercer 6, the blood thinly spreads in (the hole in) the perforated portion 34 within the thickness (height) of the perforated portion 34. Accordingly, this also makes it difficult to bring the blood 35 into the blood collection tube 31 and it is possible to prevent the blood 35 from entering the sample 36.

In addition, the ventilation passage 9 is formed in the first piercer 6 and, when the first piercer 6 penetrates the perforated portion 34, the internal space 45 of the blood collection tube 31 is spatially connected to and communicates with the outside of the blood collection tube 31 via the ventilation passage 9. Accordingly, the ventilation passage 9 of the first piercer 6 is capable of bringing the internal space 45 to atmospheric pressure.

Further, the first piercer 6 is present inside the second piercer 7 when the internal space 45 of the blood collection tube 31 is spatially connected to the outside of the blood collection tube 31. Accordingly, it is possible to prevent the sample 36 from entering and adhering to the second piercer 7 even if the sample 36 in the blood collection tube 31 is scattered during perforation by the first piercer 6.

As a result, it is possible to prevent the dripping of the sample from occurring in the second piercer 7. Then, it is possible to prevent the automatic analysis device 2 from performing liquid surface detection with respect to a dripped sample 36b. Here, an extremely slight gap is present between the second piercer 7 and the first piercer 6 as described above. However, it is possible to more reliably prevent the sample 36 from entering the second piercer 7 by setting the size of this gap to, for example, the minimum required size for the first piercer 6 to be relatively movable in the second piercer 7.

Further, perforation is performed first by the first piercer 6, which is smaller in diameter than the second piercer 7, and thus it is possible to suppress the elastic restoring force at a time of perforation as compared with, for example, a case where the piercer 41 performs perforation alone as in the related art.

In addition, the first piercer 6 has the tapered portion 8, which becomes thin toward the tip, and thus the first piercer 6 is capable of piercing the perforated portion 34 more reliably. Accordingly, it is possible to prevent the perforated portion 34 from being separated from a cap main body 33 and dropping (falling) into the blood collection tube 31.

Further, as a result, it is possible to prevent deterioration in inspection accuracy with the sampling mechanism 1 of the present embodiment. Then, it is possible to forestall originally unnecessary re-inspection resulting from poor inspection accuracy attributable to false sample liquid surface detection in the sampling mechanism 1.

In addition, the tapered surface 12, which narrows downward, is formed at the tip of the second piercer 7 in the sampling mechanism 1 of the present embodiment. Accordingly, piercing of the perforated portion 34 is easy also with regard to the second piercer 7 and the elastic restoring force at a time of penetration can be suppressed.

It should be noted that the invention can be variously modified without departing from the scope of the invention. For example, the lower end of the second piercer 7 can be formed into a pointed cylindrical shape with a diagonally processed tip as in the case of the piercer of the related art.

In addition, the tip of the first piercer 6 may have, for example, a pyramid shape or the shape of a pointed thin plate such as a cutter blade. Further, the position of arrival of the first piercer 6 in the perforated portion 34 may be an eccentric position instead of the middle portion on the upper surface of the perforated portion 34. In this case, the distribution of the stress that is generated in the perforated portion 34 is not uniform when the first piercer 6 enters by piercing from the eccentric position to the perforated portion 34, and thus the first piercer 6 is capable of entering the perforated portion 34 with ease and the perforated portion 34 becomes unlikely to fall from the cap main body 33.

In addition, application is possible to the blood collection tube 31 of various types. In this case, application is also possible to the cap 32 of various types although the blood collection tube 31 having the cap 32 and the cap main body 33 and the perforated portion 34 constituting the cap 32 has been described as an example in the embodiment described above. For example, although not illustrated, the cap 32 may be of a type that has a film body and a rubber part (perforated portion) in the upper portion thereof that is penetrated by the first piercer 6 and the second piercer 7.

### EXPLANATIONS OF LETTERS OR NUMERALS

- 1: SAMPLING MECHANISM
- 2: AUTOMATIC ANALYSIS DEVICE
- 3: COMPOSITE PIERCER
- 6: FIRST PIERCER
- 7: SECOND PIERCER
- 9: VENTILATION PASSAGE
- 11: OPENING
- 31: BLOOD COLLECTION TUBE
- 32: CAP
- 34: PERFORATED PORTION
- 35, 35a, 35b: BLOOD
- 36, 36a, 36b: SAMPLE
- 42: PROBE

## Claims

1. A sampling mechanism (1) for inserting a tubular perforating body (3) into a plug body (32) of a sampled container (31) accommodating a sample (36) and performing sample sampling by passing a probe (42) through the perforating body (3) with the plug body (32) penetrated, the sampling mechanism (1) comprising the perforating body (3),
wherein the perforating body (3) has:
a first perforating body (6) inserted into the plug body (32) and penetrating the plug body (32) with a tip closed (8); and
a second perforating body (7) formed in a tubular shape, disposed outside the first perforating body (6), inside of which the first perforating body (6) is slidably mounted with extremely slight gap, and approaching the plug body (32) together with the first perforating body (6) by causing the first perforating body (6) to protrude downward, reaching the plug body (32) after the first perforating body (6) reaches the plug body (32), and penetrating the plug body (32) by being inserted into the plug body (32), and
the probe (42) is passed through the second perforating body (7) remaining after retraction of the first perforating body (6).

2. The sampling mechanism (1) according to claim 1, wherein the first perforating body (6) has a ventilation passage (9) open toward a side portion at a part reaching an internal space (45) of the sampled container (31) and allowing an inside and an outside of the sampled container (31) to communicate with each other.

3. An automatic analysis device (2) comprising the sampling mechanism (1) according to claim 1 or 2.

4. A sampling method for inserting a perforating body (3) into a plug body (32) of a sampled container (31) accommodating a sample (36) and performing sample sampling by passing a probe (42) through the perforating body (3), the perforating body (3) having a first perforating body (6) having a closed tip (8) and a second perforating body (7) formed in a tubular shape, disposed outside the first perforating body (6), inside of which the first perforating body (6) is slidably mounted with extremely slight gap, the sampling method comprising:
a step of disposing the second perforating body (7) outside the first perforating body (6), causing the first perforating body (6) to protrude below the second perforating body (7), and causing the first perforating body (6) and the second perforating body (7) to approach the plug body (32) together;
a step of inserting the first perforating body (6) into the plug body (32);
a step of causing the second perforating body (7) to reach the plug body (32) after the first perforating body (6) reaches the plug body (32) and inserting the second perforating body (7) into the plug body (32);
a step of causing the first perforating body (6) and the second perforating body (7) to penetrate the plug body (32);
a step of retracting the first perforating body (6); and
a step of passing the probe (42) through the remaining second perforating body (7).

5. The sampling method according to claim 4, wherein the first perforating body (6) is provided with a ventilation passage (9) open toward a side portion at a part reaching an internal space (45) of the sampled container (31) and the first perforating body (6) is retracted after the ventilation passage (9) allows an inside and an outside of the sampled container (31) to communicate with each other.

## Patentansprüche

1. Probennahmemechanismus (1) zum Einführen eines rohrförmigen Perforationskörpers (3) in einen Stopfenkörper (32) eines eine Probe (36) aufnehmenden Probennahmebehälters (31), und zum Durchführen einer Probennahme durch Hindurchführen einer Sonde (42) durch den Perforationskörper (3), während der Stopfenkörper (32) durchdrungen ist, wobei der Probennahmemechanismus (1) den Perforationskörper (3) aufweist,
wobei
der Perforationskörper (3) aufweist:
einen in den Stopfenkörper (32) eingeführten und den Stopfenkörper (32) mit einer geschlossenen Spitze (8) durchdringenden ersten Perforationskörper (6); und
einen rohrförmig ausgebildeten, außerhalb des ersten Perforationskörpers (6) angeordneten zweiten Perforationskörper (7), in dessen Inneren der erste Perforationskörper (6) mit äußerst geringem Spalt verschiebbar eingebaut ist und der sich zusammen mit dem ersten Perforationskörper (6) dem Stopfenkörper (32) nähert, indem der erste Perforationskörper (6) veranlasst wird, nach unten vorzustehen, der den Stopfenkörper (32) erreicht, nachdem der erste Perforationskörper (6) den Stopfenkörper (32) erreicht hat, und der den Stopfenkörper (32) durchdringt, indem er in den Stopfenkörper (32) eingeführt wird, und
die Sonde (42) durch den nach Rückzug des ersten Perforationskörpers (6) verbleibenden zweiten Perforationskörper (7) geführt wird.

2. Probennahmemechanismus (1) nach Anspruch 1, wobei der erste Perforationskörper (6) einen Belüftungskanal (9) aufweist, der zu einem Seitenabschnitt an einem Teil offen ist, der einen Innenraum (45) des Probennahmebehälters (31) erreicht, und ermöglicht, dass ein Inneres und ein Äußeres des Probennahmebehälters (31) miteinander kommunizieren.

3. Automatische Analysevorrichtung (2), aufweisend den Probennahmemechanismus (1) nach Anspruch 1 oder 2.

4. Probennahmeverfahren zum Einführen eines Perforationskörpers (3) in einen Stopfenkörper (32) eines eine Probe (36) aufnehmenden Probennahmebehälters (31), und zum Durchführen einer Probennahme durch Hindurchführen einer Sonde (42) durch den Perforationskörper (3), wobei der Perforationskörper (3) einen ersten Perforationskörper (6) mit einer geschlossenen Spitze (8) und einen rohrförmig ausgebildeten, außerhalb des ersten Perforationskörpers (6) angeordneten zweiten Perforationskörper (7) aufweist, innerhalb dessen der erste Perforationskörper (6) mit extrem geringem Spalt verschiebbar angebracht ist, wobei das Probennahmeverfahren aufweist:
einen Schritt des Anordnens des zweiten Perforationskörpers (7) außerhalb des ersten Perforationskörpers (6), Veranlassens des ersten Perforationskörpers (6), unterhalb des zweiten Perforationskörpers (7) vorzustehen, und Veranlassens des ersten Perforationskörpers (6) und des zweiten Perforationskörpers (7), sich gemeinsam dem Stopfenkörper (32) zu nähern;
einen Schritt des Einführens des ersten Perforationskörpers (6) in den Stopfenkörper (32);
einen Schritt des Veranlassens des zweiten Perforationskörpers (7), den Stopfenkörper (32) zu erreichen, nachdem der erste Perforationskörper (6) den Stopfenkörper (32) erreicht hat, und Einführens des zweiten Perforationskörpers (7) in den Stopfenkörper (32);
einen Schritt des Veranlassens des ersten Perforationskörpers (6) und des zweiten Perforationskörpers (7), den Stopfenkörper (32) zu durchdringen;
einen Schritt des Zurückziehens des ersten Perforationskörpers (6); und einen Schritt des Hindurchführens der Sonde (42) durch den verbleibenden zweiten Perforationskörper (7).

5. Probennahmeverfahren nach Anspruch 4, wobei der erste Perforationskörper (6) mit einem Belüftungskanal (9) vorgesehen ist, der zu einem Seitenabschnitt an einem Teil offen ist, der einen Innenraum (45) des Probennahmebehälters (31) erreicht, und der erste Perforationskörper (6) zurückgezogen wird, nachdem der Belüftungskanal (9) ermöglicht hat, dass ein Inneres und ein Äußeres des Probennahmebehälters (31) miteinander kommunizieren.

## Revendications

1. Mécanisme d'échantillonnage (1) destiné à insérer un corps de perforation tubulaire (3) dans un corps de bouchon (32) d'un contenant d'échantillonnage (31) recevant un échantillon (36) et à réaliser un échantillonnage d'échantillon en faisant passer une sonde (42) à travers le corps de perforation (3) avec pénétration dans le corps de bouchon (32), le mécanisme d'échantillonnage (1) comprenant le corps de perforation (3), dans lequel
le corps de perforation (3) présente :
un premier corps de perforation (6) inséré dans le corps de bouchon (32) et pénétrant dans le corps de bouchon (32) avec une pointe fermée (8) ; et
un second corps de perforation (7) formé dans une forme tubulaire, disposé à l'extérieur du premier corps de perforation (6), à l'intérieur duquel le premier corps de perforation (6) est monté de manière coulissante avec un vide extrêmement léger, et s'approchant du corps de bouchon (32) conjointement avec le premier corps de perforation (6) en amenant le premier corps de perforation (6) à faire saillie vers le bas, atteignant le corps de bouchon (32) une fois que le premier corps de perforation (6) a atteint le corps de bouchon (32), et pénétrant dans le corps de bouchon (32) en étant inséré dans le corps de bouchon (32), et
la sonde (42) est passée à travers le second corps de perforation (7) restant après la rétraction du premier corps de perforation (6).

2. Mécanisme d'échantillonnage (1) selon la revendication 1, dans lequel le premier corps de perforation (6) présente un passage de ventilation (9) ouvert vers une portion latérale au niveau d'une partie atteignant un espace interne (45) du contenant d'échantillonnage (31) et permettant à l'intérieur et l'extérieur du contenant d'échantillonnage (31) de communiquer l'un avec l'autre.

3. Dispositif d'analyse automatique (2) comprenant le mécanisme d'échantillonnage (1) selon la revendication 1 ou 2.

4. Procédé d'échantillonnage destiné à insérer un corps de perforation (3) dans un corps de bouchon (32) d'un contenant d'échantillonnage (31) recevant un échantillon (36) et à réaliser un échantillonnage d'échantillon en faisant passer une sonde (42) à travers le corps de perforation (3), le corps de perforation (3) présentant un premier corps de perforation (6) ayant une pointe fermée et un second corps de perforation (7) formé dans une forme tubulaire, disposé à l'extérieur du premier corps de perforation (6), à l'intérieur duquel le premier corps de perforation (6) est monté de manière coulissante avec un vide extrêmement léger, le procédé d'échantillonnage comprenant :
une étape destinée à disposer le second corps de perforation (7) à l'extérieur du premier corps de perforation (6), amenant le premier corps de perforation (6) à faire saillie au-dessous du second corps de perforation (7), et amenant le premier corps de perforation (6) et le second corps de perforation (7) à s'approcher ensemble du corps de bouchon (32) ;
une étape destinée à insérer le premier corps de perforation (6) dans le corps de bouchon (32) ;
une étape destinée à amener le second corps de perforation (7) à atteindre le corps de bouchon (32) une fois que le premier corps de perforation (6) a atteint le corps de bouchon (32) et à insérer le second corps de perforation (7) dans le corps de bouchon (32) ;
une étape destinée à amener le premier corps de perforation (6) et le second corps de perforation (7) à pénétrer dans le corps de bouchon (32) ;
une étape destinée à rétracter le premier corps de perforation (6) ; et
une étape destinée à faire passer la sonde (42) à travers le second corps de perforation (7) restant.

5. Procédé d'échantillonnage selon la revendication 4, dans lequel le premier corps de perforation (6) est muni d'un passage de ventilation (9) ouvert vers une portion latérale au niveau d'une partie atteignant un espace interne (45) du contenant d'échantillonnage et le premier corps de perforation (6) est rétracté une fois que le passage de ventilation (9) permet à l'intérieur et l'extérieur du contenant d'échantillonnage (31) de communiquer l'un avec l'autre.
